# EUROPÄISCHE PATENTSCHRIFT

(11) **EP 0 370 571 B1**
(45) Veröffentlichungstag und Bekanntmachung des Hinweises auf die Patenterteilung: **06.10.1993**
(21) Anmeldenummer: 89202915.8
(22) Anmeldetag: 17.11.1989
(51) Int. Cl.: A61M 5/30

(54) **Vorrichtung zur nadellosen, subkutanen Injektion von Medikamenten**
Device for needle-less subcutaneous medicament injection
Dispositif d'injection sous-cutanée de médicaments, sans aiguille

(30) Priorität: 21.11.1988 DE 3839287; 20.01.1989 DE 3901691
(43) Veröffentlichungstag der Anmeldung: 30.05.1990
(73) Patentinhaber: Holzer, Walter, Senator h.c. Dr.h.c.Ing., D-88709 Meersburg (DE)
(72) Erfinder: Holzer, Walter, Senator h.c. Dr.h.c.Ing., D-88709 Meersburg (DE)
(74) Vertreter: Riebling, Peter, Dr.-Ing.

(56) Entgegenhaltungen:
- DE-A- 1 491 695
- US-A- 3 688 765
- US-A- 3 788 315

## Beschreibung

Die Erfindung betrifft eine Vorrichtung zur nadellosen Injektion eines flüssigen Medikaments mit Hilfe einer das Medikament in eine Hohlraum enthaltenden Ampulle, die eine Frontplatte mit wenigstens einer Abgabeöffnung aufweist, wobei der Hohlraum an der Rückseite mit einer verformbaren Membran abgeschlossen ist, wobei ferner die Ampulle lösbar in das Vorderende einer Injektionspistole einsetzbar ist, die zum Abgeben des Medikaments einen federbelasteten Stempel zur schlagartigen Anlage an der Membran der Ampulle hat.

Derartige Vorrichtungen werden insbesondere bei Massenaktionen, wie Impfungen oder ähnlichen vorbeugenden Maßnahmen, verwendet, wo oft tausende Menschen in kurzer Zeit eine Injektion erhalten müssen.

Eine Vorrichtung mit den eingangs genannten Merkmalen ist in der DE-A-1 491 965 beschrieben. Das Vorderende des Stempels ist zylindrisch ausgebildet. Beim Auslösen des Stempels bildet sich ein Ringwulst der Membran rings um das Vorderende des Stempels, wobei die Gefahr besteht, dass die Membran reisst oder platzt, was natürlich nachteilig ist.

Hiervon ausgehend liegt der Erfindung die Aufgabe zugrunde, eine Vorrichtung zur nadellosen Injektion eines Medikaments mit den Merkmalen des Oberbegriffs von Patentanspruch 1 so auszugestalten, daß beim Aufschlagen des Stempels auf die Membran deren Platzen verhindert wird.

Die Lösung dieser Aufgabe gelingt erfindungsgemäss dadurch, daß das Vorderende des Stempels eine der Form der Membran angepasste Hohlform aufweist.

Bevorzugt wird es, wenn am Vorderende des Stempels eine entsprechend geformte Hülse befestigt ist. Durch ein solches formschlüssiges Umschließen der Membran beim Entleeren der Ampulle wird ein Platzen der Membran verhindert.

Die Erfindung wird im folgenden anhand von Zeichnungen näher erläutert, wobei sich weitere Merkmale und Vorteile ergeben.

Es zeigt:
- Figur 1 bis Figur 4:: Ausführungsbeispiele erfindungsgemässer Ampullen;
- Figur 5 bis Figur 7:: die Anordnung einer Ampulle in einer Injektionspistole in verschiedenen Phasen;
- Figur 8 und Figur 9:: die Aufbewahrung der Ampullen an einer Platte mit Aufnahmelöchern;
- Figur 10:: die Anordnung von Codierungen an der Frontplatte einer Ampulle;
- Figur 11:: ein Ausführungsbeispiel möglicher Codierung;
- Figur 12:: Die Anordnung weiterer Codierungen an der Aufnahmeplatte, die als Verpackung dient.

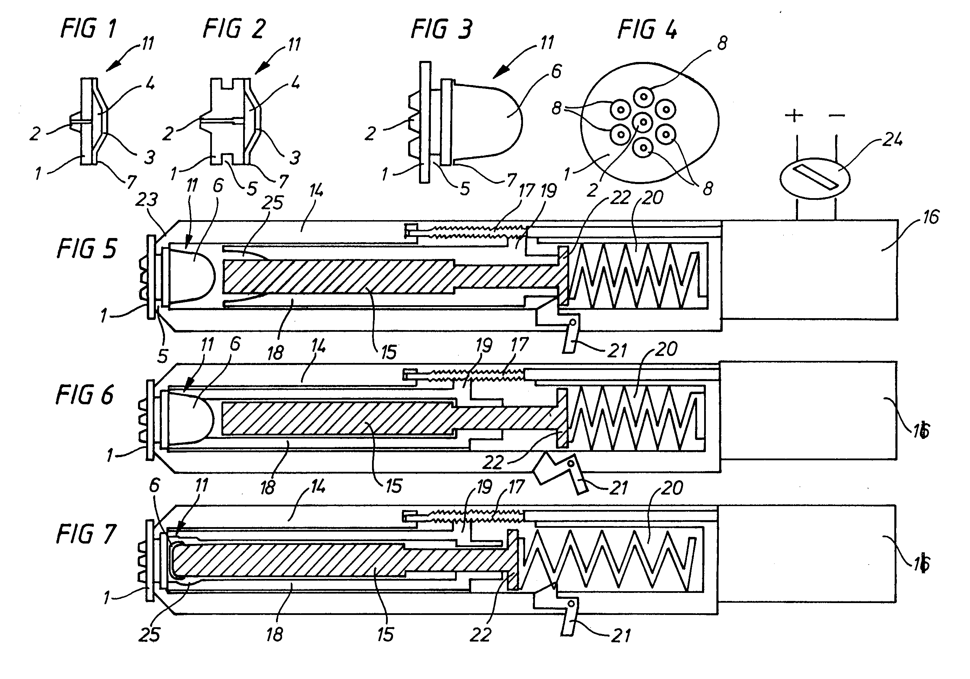

Die Figur 1 und Figur 2 stellen eine einfachste Form dar. Diese Ampulle besteht nur aus einer Frontplatte (1) mit einer Düse (2) und einer Membran (3), in der das Medikament (4) als Flüssigkeit enthalten ist.

Figur 3 zeigt eine Weiterausbildung des Erfindungsgedankens. Die Frontplatte (1) ist wesentlich größer als der Durchmesser des dahinter liegenden Rollbalges (6), der am Rand (7) mit der Frontplatte (1) durch Schweißen oder Kleben verbunden ist.

Die Frontplatte (1) trägt in diesem Ausführungsbeispiel nach den Figuren 3 und 4 sechs Düsen (8), die kreisförmig um die zentrale Düse (2) angeordnet sind.

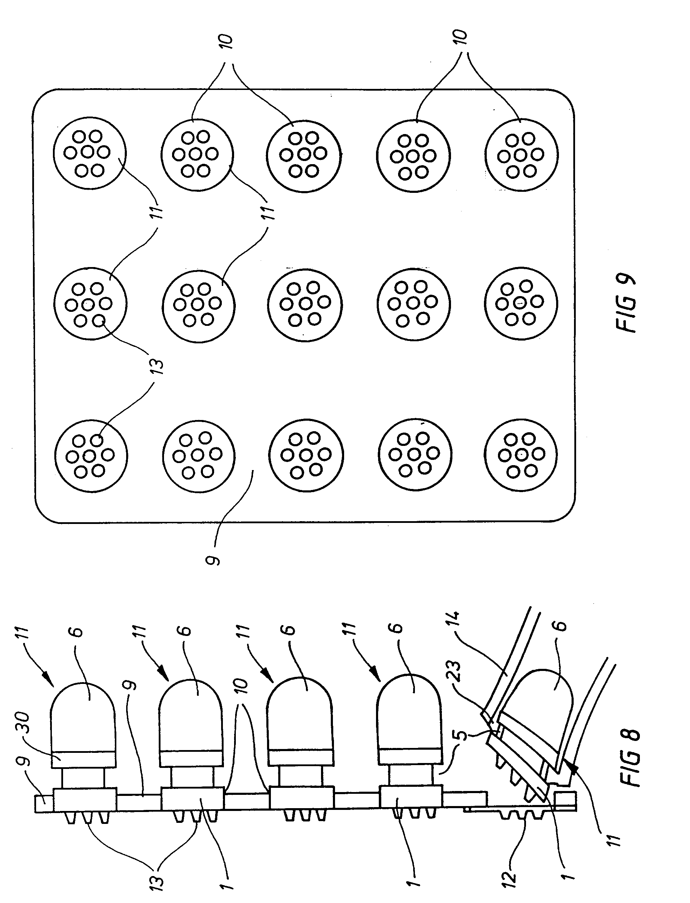

Figur 8 und Figur 9 zeigen als Beispiel eine Platte (9) mit fünfzehn Aufnahmelöchern (10), in welche die Ampullen (11) eingesetzt sind.

Auf der Vorderseite der Platte (9) befindet sich die Folie (12), die vorteilhafterweise unter Vacuum auf die Platte (9) und die Ampullen (11) aufgebracht wird. Die Folie (12) hält die Ampullen (11) fest und verschließt dabei die Düsen (13).

Beim Herauslösen der Ampullen bleibt die Folie (12) an der Platte (9), wie in Figur 8 gezeigt, und stört nicht die weitere Handhabung.

Figur 8 zeigt auch wie einfach das Entnehmen der Ampulle (11) aus der als Magazin dienenden Lochplatte (9) ist.

Mit der Injektionspistole (14) wird die Ampulle (11) in ihrer Nut (5) aufgenommen mit mit einer leichten Kippbewegung, wie in Figur 8 gezeigt, von der Folie (12) abgezogen.

Besondere Bedeutung kommt auch einer erfindungsgemäßen Injektionspistole (14) zu, wie in den Figurten 5,6 und 7 in verschiedenen Phasen dargestellt.

Figur 5 zeigt die Pistole (14) in gespanntem Zustand, Dabei befindet sich der Stempel (15) in der rückwärtigen Lage, in welche er durch den Getriebemotor (16) mit seiner Gewindespindel (17) und der Hülse (18) mit ihrem Gegengewinde (19) gebracht wurde. Dabei wandert das Gegengewinde (19) mit der Hülse (18) in Richtung der Feder (20) und nimmt die Federplatte (22) des Stempels (15) mit, die ihrerseits die Feder (20) spannt, bis der Abzug (21) hinter der Federplatte (22) einrastet.

Die Ampulle (11) ist mit ihrer Nut (5) in der Aufnahme (23) gehalten.

Figur 6 zeigt die Injektionspistole (14) schußbereit. Vorher wurde die Hülse (18) vom Getriebemotor (16), der durch einen kleinen Wendeschalter (24) gewendet werden kann, in die dargestellte Lage gebracht. Die Hülse (18) umschließt jetzt formschlüssig mit ihrer Hohlform (25) die Ampulle (11) bzw. den Rollbalg (6) und verhindert dadurch ein Platzen beim Aufschlagen des Stempels (15). Der Abzug (21) ist bereits entriegelt dargestellt und der Stemnpel (15) kann jetzt unter der Kraft der Feder (20) nach vorne schnellen und verformt dabei die elastische Membran (3,6) der Ampulle (11), wie in Figur 7 dargestellt. Es ist deutlich die Verformung des Rollbalges (6) zu erkennen, der sich unter dem Druck des Stempels (15) nach innen gestülpt und eingerollt hat. Der Motor kann nun wieder von dem Wendeschalter (24) umgeschaltet werden und bringt wieder die Injektionspistole (14) in die Lage Figur 5. Die verbrauchte Ampulle (11) kann nun herausgenommen und eine neue aus der Platte (9) entnommen werden.

Die dargestellten Ausführungen sind nur als schematische Beispiele zu verstehen, wobei wesentliche Einzelheiten den Zeichnungen zu entnehmen sind, z. B. ist der Wendeschalter (24) als Kombination von Endschaltern denkbar und auch das automatische Auswerfen der gebrauchten Ampullen kann erfindungsgemäß vorgesehen werden. In diesem Sinn sind die angeführten Beispiele nicht als einschränkend aufzufassen.

Um auch die Vorrichtung zur nadellosen, subkutanen Injektion bei Diabetikern anwenden zu können, ist es vorteilhaft, wenn die Ampullen und/oder die Verpackung mit einer Codierung versehen werden.

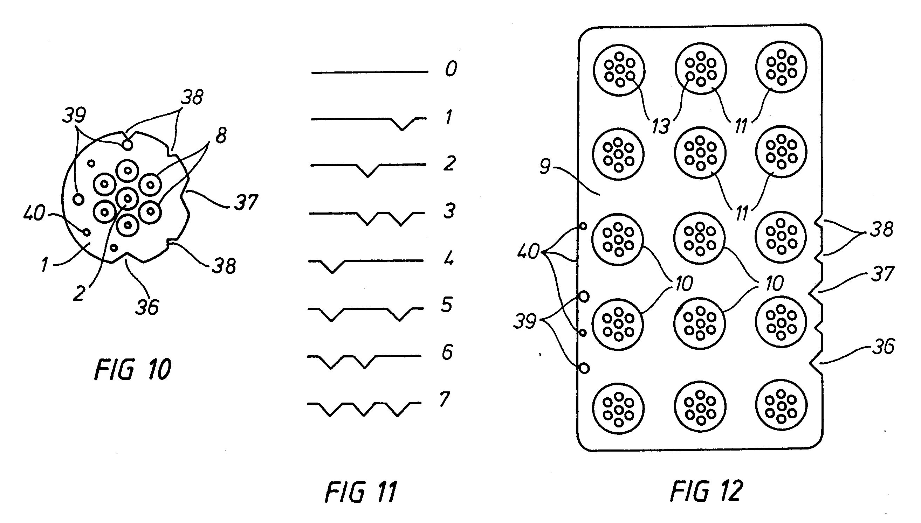

Für die Anwendung des erfindungsgemäßen Verfahrens bei Diabetes ist es erforderlich, etwa zwischen einer Einheit und maximal vierzig Einheiten zu dosieren. Es wäre daher denkbar, einen Satz von abgestuften Ampullen bereitzustellen, z. B. mit
1,2,4,8,16,24,32 und 40 Einheiten
Diese acht verschiedenen Ampullen könnten, z. B. gemäß Figur 11, digital durch Kerben numeriert werden. An der Ampulle könnten diese Kerben an der vorderen Platte (1) angebracht werden, wie Figur 10 zeigt.

Durch Erhöhung der Kerben, z. B. auf sechs Stellen, ist es auch möglich, nicht nur eine Numerierung, sondern auch digital die tatsächliche Anzahl der Einheiten zu codieren. Die in Figur 10 dargestellte Codierung würde digital bedeuten, daß die an der ersten Stelle die Kerbe (36) und an der dritten Stelle die Kerbe (37) eine Einheitenanzahl von eins plus vier, also fünf Einheiten bedeuten. Die kleinen Kerben (38) dienen als Orientierungshilfe beim Abtasten.

Die gleiche Codierung mit den Kerben (36) und (37) kann auch am Rand der Platte (9) nach Figur 12 angebracht werden, um den Inhalt der Verpackung erkennen zu können.

Die in Figur 10 und Figur 12 angedeuteten Erhöhungen (39) und (40) erfüllen den gleichen Zweck, wie die Kerben (36,37,38), sind aber der traditionellen Blindenschrift vergleichbar.

Das erfindungsgemäße Verfahren ist selbstverständlich keineswegs auf die schematischen Beispiele und den gezeigten Formen beschränkt. Es läßt sich erfindungsgemäß genauso für bisher gebräuchliche Ampullen verwenden.

Die farbliche Codierung kann auf den schwarz-weißen Patentzeichnungen nicht dargestellt werden, aber erfindungsgemäß wäre es zweckmäßig, z. B. die Platte (1) der Ampullen nach den Figuren 1 bis 4, aber auch z. B. die Platte (9) nach den Figuren 8,9 und Figur 12 in den entsprechenden Codierungsfarben zu kennzeichnen.

Ebenso wäre es erfindungsgemäß eine zusätzliche Ausstattung, mit einem computerlesbaren Strichcode oder anderer gebräuchlicher Codierungsverfahren möglich,

Das vorgeschlagene Verfahren schließt nicht nur menschliches Versagen und Irrtümer bei der Einstellung der Dosierung aus, sondern erleichtert die Handhabung und erhöht die Sicherheit bei der Behandlung von Kranken.

Kostenmäßig ist vor allem die Farbcodierung ohne jeden zusätzlichen Aufwand verbunden, aber auch die Copierung mit Kerben und ähnlichen Kennzeichnungen können im Zuge einer vollautomatischen Produktion ohne wesentlichen Aufwand vorgenommen werden.

### ZEICHNUNGS-LEGENDE

- 1: Frontplatte
- 2: Düse
- 3: Membrane
- 4: Medikament
- 5: Nut
- 6: Rollbalg
- 7: Rand
- 8: Düse
- 9: Platte
- 10: Aufnahmelöcher
- 11: Ampulle
- 12: Folie
- 13: Düse
- 14: Injektionspistole
- 15: Stempel
- 16: Getriebemotor
- 17: Gewindespindel
- 18: Hülse
- 19: Gegengewinde
- 20: Feder
- 21: Abzug
- 22: Federplatte
- 23: Aufnahme
- 24: Wendeschalter
- 25: Hohlform
- 36: Kerbe
- 37: Kerbe
- 38: Kerbe
- 39: Erhöhung
- 40: Erhöhung

## Patentansprüche

1. Vorrichtung zur nadellosen Injektion eines flüssigen Medikaments (4) mit Hilfe einer das Medikament in einem Hohlraum enthaltenden Ampulle (11), die eine Frontplatte (1) mit wenigstens einer Abgabeöffnung (2,8,13) aufweist, wobei der Hohlraum an der Rückseite mit einer verformbaren Membran (3,6) abgeschlossen ist, wobei ferner die Ampulle (11) lösbar in das Vorderende einer Injektionsspistole (14) einsetzbar ist, die zum Abgeben des Medikaments einen federbelasteten Stempel (15) zur schlagartigen Anlage an der Membran (3,6) der Ampulle (11) hat, **dadurch gekennzeichnet,** daß das Vorderende des Stempels (15) eine der Form der Membran (3,6) angepasste Hohlform (25) aufweist.

2. Vorrichtung nach Anspruch 1, **dadurch gekennzeichnet,** daß am Vorderende des Stempels (15) eine entsprechend geformte Hülse (18) befestigt ist.

3. Vorrichtung nach Anspruch 1 oder 2, **dadurch gekennzeichnet,** daß zumindest einige der Öffnungen (2,8,13) kreisförmig angeordnet und als Düsen ausgebildet sind.

4. Vorrichtung nach den Ansprüchen 1 bis 3, **dadurch gekennzeichnet,** daß die Ampulle (11) mit einer Nut (5) zur Halterung in eine klauenförmige Aufnahme (23) der Injektionspistole (14) einsetzbar ist.

5. Vorrichtung nach Anspruch 1, **dadurch gekennzeichnet,** daß die Ampulle (11) in eine gelochte Aufnahmeplatte (9) einsetzbar ist, wobei die Aufnahmeplatte (9) an der Außenseite eine klebende Folie (12) aufweist, welche die Ampullen (11) in der Frontplatte (1) in Aufnahmelöchern (10) festhält und hierbei die Düsen (2,8,13) verschließt.

6. Vorrichtung nach Anspruch 1, **dadurch gekennzeichnet,** daß die Injektionspistole (14) mit einem Getriebemotor (16) zum Spannen des Stempels (15) versehen ist.

7. Vorrichtung nach Anspruch 1, **dadurch gekennzeichnet,** daß jede Ampulle (11) je nach Inhalt und Wirkstoff farblich codiert gekennzeichnet ist.

8. Vorrichtung nach Anspruch 1, **dadurch gekennzeichnet,** daß die Ampullen (11) je nach Inhalt abtastbar über Kerben (36,37,38) codiert sind.

9. Vorrichtung nach Anspruch 1, **dadurch gekennzeichnet,** daß die Ampullen (11) in Blindenschrift codiert sind.

10. Vorrichtung nach Anspruch 1, **dadurch gekennzeichnet,** daß die Ampullen (11) abtastbar codiert sind, wobei die abtastbare Codierung digitale Informationen über Konzentration, Menge und/oder Wirkstoffeinheiten gibt.

11. Vorrichtung nach Anspruch 1, **dadurch gekennzeichnet,** daß die Ampullen (11) und die Platte (9) der Verpackung mit gleichartigem Farb- und/oder abtastbaren Codierungen versehen sind.

## Claims

1. Device for needleless injection of a liquid drug (4) by means of an ampoule (11) which contains the drug in a cavity and which has a front plate (1) with at least one discharge opening (2, 8, 13), wherein the cavity is closed on the rear side with a deformable diaphragm (3, 6), wherein further the ampoule (11) can be inserted releasably in the front end of an injection gun (14) which for discharge of the drug has a spring-loaded plunger (15) for sudden abutment against the diaphragm (3, 6) of the ampoule (11), characterised in that the front end of the plunger (15) has a hollow shape (25) adapted to the shape of the diaphragm (3, 6).

2. Device according to claim 1, characterised in that attached to the front end of the plunger (15) is a correspondingly shaped sleeve (18).

3. Device according to claim 1 or 2, characterised in that at least some of the openings (2, 8, 13) are arranged in a circle and constructed as nozzles.

4. Device according to claims 1 to 3, characterised in that the ampoule (11) can be inserted by a groove (5) for support in a claw-shaped holder(23) of the injection gun (14).

5. Device according to claim 1, characterised in that the ampoule (11) can be inserted in a perforated receiving plate (9), wherein the receiving plate (9) has on the outside an adhesive film (12) which holds the ampoules (11) fast in the front plate (1) in receiving holes (10) and in doing so closes the nozzles (2, 8, 13).

6. Device according to claim 1, characterised in that the injection gun (14) is provided with a gear motor (16) for clamping the plunger (15).

7. Device according to claim 1, characterised in that each ampoule (11) is marked with a colour code depending on contents and active ingredient.

8. Device according to claim 1, characterised in that the ampoules (11) are coded by notches (36, 37, 38) so as to be capable of being scanned depending on contents.

9. Device according to claim 1, characterised in that the ampoules (11) are coded in Braille.

10. Device according to claim 1, characterised in that the ampoules (11) are coded so as to be capable of being scanned, wherein the scannable coding gives digital information on concentration, quantity and/or units of active ingredient.

11. Device according to claim 1, characterised in that the ampoules (11) and the plate (9) of the package are provided with analogous colour and/or scannable coding.

## Revendications

1. Dispositif pour l'injection sans aiguille d'un médicament liquide (4) à l'aide d'une ampoule (11) contenant le médicament dans une cavité et comportant une plaque frontale (1) pourvue d'une ou plusieurs ouvertures de décharge (2, 8, 13), étant précisé que la cavité est fermée, à l'arrière, par une membrane déformable (3, 6), et que l'ampoule (11) peut être placée de façon détachable dans l'extrémité avant d'une seringue (14) qui comporte, en vue d'administrer le médicament, un piston (15) chargé par ressort destiné à être appliqué brusquement contre la membrane (3, 6) de l'ampoule (11), caractérisé en ce que l'extrémité avant du piston (15) présente une forme creuse (25) adaptée à la forme de la membrane (3, 6).

2. Dispositif selon la revendication 1, caractérisé en ce qu'un manchon (18) de forme correspondante est fixé à l'extrémité avant du piston (15).

3. Dispositif selon la revendication 1 ou 2, caractérisé en ce que quelques-unes au moins des ouvertures (2, 8, 13) sont disposées en cercle et sont conçues comme des injecteurs.

4. Dispositif selon les revendications 1 à 3, caractérisé en ce que l'ampoule (11) pourvue d'une rainure (5) peut être placée, en vue de la fixation, dans un logement en forme de griffe (23) de la seringue (14).

5. Dispositif selon la revendication 1, caractérisé en ce que l'ampoule (11) peut être placée dans une plaque de logement perforée (9), ladite plaque de logement (9) comportant, sur la face extérieure, une feuille adhésive (12) qui maintient dans des trous de logement (10) les ampoules (11) prévues dans la plaque frontale (1), et obture ainsi les injecteurs (2, 8, 13).

6. Dispositif selon la revendication 1, caractérisé en ce que la seringue (14) est pourvue d'un moto-réducteur (16) destiné à tendre le piston (15).

7. Dispositif selon la revendication 1, caractérisé en ce que chaque ampoule (11) est marquée à l'aide d'un codage coloré en fonction de son contenu et de son agent.

8. Dispositif selon la revendication 1, caractérisé en ce que les ampoules (11) sont codées, suivant leur contenu, de façon palpable grâce à des encoches (36, 37, 38).

9. Dispositif selon la revendication 1, caractérisé en ce que les ampoules (11) sont codées en braille.

10. Dispositif selon la revendication 1, caractérisé en ce que les ampoules (11) sont codées de façon palpable, le codage palpable donnant des informations numériques sur la concentration, la quantité et/ou les unités d'agent.

11. Dispositif selon la revendication 1, caractérisé en ce que les ampoules (11) et la plaque (9) de l'emballage sont pourvues d'un même codage coloré et/ou palpable.
